# EUROPEAN PATENT SPECIFICATION

(11) **EP 3 418 320 B1**
(45) Date of publication and mention of the grant of the patent: **02.09.2020**
(21) Application number: 17753323.9
(22) Date of filing: 17.02.2017
(51) Int. Cl.: A61K 8/02, A61K 8/25, A61K 8/895, A61Q 1/02, A61Q 17/04, A61Q 19/00, C08K 3/36, C08G 77/20, C08L 83/04, C09D 183/04, C08K 9/06

(54) **SILICA-COATED SILICONE RUBBER PARTICLES AND COSMETIC**
MIT SILIKA BESCHICHTETE SILIKONKAUTSCHUKPARTIKEL UND KOSMETIK
PARTICULES DE CAOUTCHOUC SILICONÉ REVÊTUES DE SILICE ET PRODUIT COSMÉTIQUE

(30) Priority: 19.02.2016 JP 2016029697
(43) Date of publication of application: 26.12.2018
(73) Proprietor: Wacker Chemie AG, 81737 München (DE)
(72) Inventor: IGARASHI Kenji, Chikusei-shi Ibaraki 300-4522 (JP)
(74) Representative: Mieskes, Klaus Theoderich
(86) International application number: PCT/JP2017/005942
(87) International publication number: WO 2017/142068

(56) References cited:
- EP-A1- 0 647 672
- JP-A- H1 036 219
- JP-A- H04 168 117
- JP-A- H07 102 075
- JP-A- H10 175 816
- US-A1- 2015 024 015

## Description

### TECHNICAL FIELD

The present invention relates to a method for manufacturing a silica-coated silicone rubber particle having advantageous properties such as having an almost truly spherical shape to exhibit less blocking.

### BACKGROUND ART

Since silicone rubber particles have high viscoelasticity, light scattering, transmittance, and oil absorption properties due to the wide surface area, they are widely used in industrial fields such as cosmetics, synthetic resin materials, and synthetic rubber materials.

Formulating silicone powder in cosmetics to be applied to skin is under consideration for improving the sense of touch after application, obtaining smoothness in appearance, and absorbing sebum to prevent smeared makeup. Cosmetics have various forms such as oil, water-in-oil, and oil-in-water depending on uses. In particular, the oil-in-water form tends to be preferred in recent years, for the reason that it is excellent in the sense of touch after application. However, since silicone powder is hydrophobic by nature, an ionic or non-ionic organic surfactant or the like needs to be used to provide hydrophilicity to the silicone powder so that it can be successfully formulated into the oil-in-water type cosmetics. For example, in Patent Literature 1 and Patent Literature 2, water-dispersible silicone rubber particles including crosslinked silicone particles, a surfactant, and water have been proposed.

Among these water-dispersible silicone rubber particles, a water-dispersible silicone rubber particle which is dispersed with a non-ionic surfactant of polyoxyalkylene alkyl ethers is widely used from the viewpoint of skin irritation.

However, the shape of the particle prepared with these surfactants is not necessarily a true sphere. Also, the particle size distribution is often broad. Furthermore, the surface of the particle may not be successfully coated. For these reasons and others, blocking and secondary aggregation are likely to be caused.

Also, known silicone rubber particles are excellent in the sense of intimate contact with skin after application when they are soft. However, soft silicone rubber particles deform when applied, causing the contact area with each other to be increased and the frictional resistance between particles to increase. Therefore, there has been a problem that extensibility thereof decreases or that particles gather and become residues to come out.

Thus, known silicone rubber particles cannot sufficiently exert the properties of the particles when they are adopted for their applications. Accordingly, there has been a problem that the use applications are limited, or that demands on the particles cannot be sufficiently met.

In addition, the cohesion among particles mainly attributable to surfactant components causes an unfavorable state when used such as blocking in which the smooth movement among particles is inhibited resulting in the decrease in fluidity and the generation of solid lumps. For solving the problem of blocking, a product obtained by mixing an inorganic filler such as silica to a silicone rubber particle after the silicone rubber particle has been produced is commercially available. However, for such a product, the process of adding silica needs to be added. Thus, there has been a problem that the production process is complicated.

Furthermore, these surfactants excessively present in a medium are sometimes concentrated during the drying process after the application to skin thereby to develop a liquid crystal layer, with the result that the senses of thickening and stickiness may sometimes be expressed. Thus, there has also been a problem that the effect of the silicone rubber particle originally formulated for the purpose of reducing stickiness is reduced.

Moreover, since a highly hydrophilic surfactant component is used for dispersing particles in an aqueous phase, there has also been an adverse effect that a mixture of sweat and sebum causes the particle layer formed on skin to be in disorder due to the action of the surfactant, with the result that the appearance of makeup is likely to be smeared.

Also, although an example of the surfactant component most commonly used in the art includes alkyl polyethers having an alkyl group with 12 to 15 carbon atoms, these surfactants are designated as a chemical which can have an adverse effect on the environment, and obligated to conform to the emission amount of PRTR (Pollutant Release and Transfer Register). Thus, the selection of these surfactants is limited.

Furthermore, Patent Literature 3 discloses the method of obtaining a silicone particle by using a silica particle in place of known surfactants to emulsify a silicone component.

However, the shape and attribute of the particle have not specifically been described. Also, a specific method for industrially obtaining these has not been disclosed. Furthermore, a specific method for obtaining the properties of the silicone particle necessary for its applications have not been disclosed.

### CITATION LIST

### PATENT LITERATURE

Patent Literature 1: Japanese Patent Application Laid-Open No. Sho. 63-309565
Patent Literature 2: Japanese Patent Application Laid-Open No. Hei. 11-140191
Patent Literature 3: Japanese Patent Application Publication No. 2009-531489

### SUMMARY OF INVENTION

### TECHNICAL PROBLEM

As described above, a silicone rubber particle which is useful owing to the characteristic shape and attribute of the particle, a specific method for industrially obtaining the useful silicone rubber particle, and a specific method for obtaining the properties of the silicone rubber particle necessary for its applications have not been known.

The present invention has been made in view of the above-described circumstances, and provides a method for manufacturing a silicone rubber particle of which shape is a substantially true sphere, and which does not have blocking problem, is industrially advantageous and easily controlled in terms of a reaction, and is excellent in dispersion stability, stability during the isolation of particles, and stability of physical properties.

### SOLUTION TO PROBLEM

The present inventor intensively conducted research. As a result, the inventor has found that a silicone rubber particle which has a surface coated with a silica particle and is characteristic in the surface coverage of the silicone rubber particle by the silica particle and in the sphericity of the silicone rubber particle has superior properties when it is employed for its use applications, and the use applications of the silicone rubber particle are not limited anymore.

The inventor has also found that a water-dispersible silicone rubber particle which is industrially advantageous and easily controlled in terms of a reaction and which is excellent in dispersion stability by a method including dispersing a vinyl group-containing siloxane and a hydrogen functional group-containing siloxane in water with a silica particle, and thereafter performing an addition curing reaction through a hydrosilylation reaction with a platinum catalyst as the method for producing the silica-coated silicone rubber particle. At the same time, the inventor has found the definition and the condition of optimization for the structure and composition of a polymer necessary for obtaining the sense of tackiness and the friction reducing effect sufficient as a silicone rubber particle, oil absorption properties, and the homogenization effect of appearance by light scattering.

The present invention has been made in view of the foregoing knowledges.

Specifically, a silica-coated silicone rubber particle manufactured according to the present invention has a surface coated with a silica particle and a sphericity of 0.8 to 1.0. The silica-coated silicone rubber particle has on its surface, a coating layer of the silica particle 2.0 × 10⁻⁹ to 3.2 × 10⁻⁶ kg/m².

### ADVANTAGEOUS EFFECTS OF INVENTION

According to the emulsion obtained in the present invention, the particle diameter is, unlike that of a known emulsion obtained with a common organic surfactant, not likely to be influenced by the polarity of the oil phase to be dispersed. Furthermore, a wide range of oil components can be stably dispersed, and a dispersed body having high sphericity can be provided. Therefore, isolation can stably be performed, and a silicone rubber particle having high sphericity can be obtained.

Since the silica-coated silicone rubber particle manufactured according to the present invention can be obtained without nonionic, anionic, cationic, and amphoteric organic surfactants which are used in general, the hydrophobic property originally possessed by the silicone rubber particle, and the sense of tackiness and the friction reducing effect due to low surface tension can be utilized to the greatest extent possible.

Even when the silicone rubber particle manufactured according to the present invention has a composition for providing soft rubber, the silicone rubber particle forms on the surface of the particle a highly hard rubber layer containing the silica particle. Therefore, the silicone rubber particle can provide a rubber particle having excellent extensibility.

Also, since the silica particle exists between the silicone rubber particles and the sphericity is high, the contact surface area decreases, the friction between the particles lowers, and smooth fluidity can be obtained. As a result, blocking which is a problem peculiar to a powder product can be cancelled. Therefore, a wide range of use applications such as cosmetics and paints becomes possible. When the silicone rubber particle according to the present invention is used in cosmetics, the stickiness is reduced, and the sebum absorbing effect can be obtained. For example, the silicone rubber particle according to the present invention can be used in cosmetics such as body soaps, shampoos, milky lotions, antiperspirants, foundations, and sunscreens.

Furthermore, since the sphericity is high, the uniformity in diffuse reflection of light as a silicone particle improves. By the synergistic effect between the improved uniformity in diffuse reflection of light and the reduced blocking properties, the soft focus effect and the sense of looseness in cosmetic uses such as foundations are exerted.

In the present invention, the silica particle is used in the process of preparing the silicone rubber particle-dispersed emulsion. This provides thixotropy properties to water medium. Therefore, separation or creaming is unlikely to be caused, and high dispersion stability and preservation stability are obtained. In addition, externally adding the catalyst and other components for curing the silicone component after the preparation of the emulsion can be performed more easily than when the emulsion is prepared with an organic surfactant.

### DESCRIPTION OF EMBODIMENTS

The method for manufacturing a silica-coated silicone rubber particle according to the present invention will be described in detail below.

The silica-coated silicone rubber particle manufactured according to the present invention is a silica-coated silicone rubber particle including a silicone rubber particle and a coating layer configured to coat the surface of the silicone rubber particle and be formed from a silica particle, in which the coating amount of the coating layer is 2.0 × 10⁻⁹ to 3.2 × 10⁻⁶ kg/m², and the sphericity is 0.8 to 1.0.

The coating contains a partial coating in addition to the entire coating. Examples of the index for representing a coating degree may include the thickness of the silica layer and the result of morphological observation. It was found this time that the use of this coating amount as a more practical coating degree enables the control of the properties. It was found that, for example, even when part of the surface of the silicone rubber particle is exposed without the silica particle coating, desired properties are obtained if this coating amount is satisfied.

The rubber elasticity of the silicone rubber portion of the silica-coated silicone rubber particle according to the present invention is in the range of 5 to 95 in terms of Shore A.

The amount of the coating layer including the silica particle on the surface can be parameterized as the weight of the silica particle per unit area of the surface of the silicone rubber particle.

In the present invention, the amount of this silica coating layer can be controlled by the processing amount of the silica particle relative to the surface area calculated from the particle diameter of the silicone rubber particle. This amount is in the range of 2.0 × 10⁻⁹ to 3.2 × 10⁻⁶ kg/m². When the amount is less than 2.0 × 10⁻⁹ kg/m², blocking of the silica-coated silicone rubber particle is caused. When the amount exceeds 3.2 × 10⁻⁶ kg/m², unfavorable phenomena such as the sense of coarseness in cosmetics are caused in specific use applications due to the increased friction of the silica-coated silicone rubber particle, and the contamination with excess silica in the emulsion or after the particle has been isolated also occurs.

In the surface layer of the silicone rubber particle coated with the silica particle, cohesion acts between silica particles or between the oil component before the curing of the silicone rubber particle and the silica particle. This cohesion causes certain interfacial tension to act, with the result that a force to minimize the surface area of the dispersed oil phase acts. As a result, the shape of the emulsified silicone rubber particle becomes closer to a true sphere.

For obtaining the silicone rubber particle having a shape closer to a true sphere, the rate of the curing reaction is not excessively high. That is, if the curing reaction proceeds while the oil layer having been distorted by shear force during emulsification and dispersion is returning to a spherical shape by the interfacial tension on the surface layer, intended truly spherical properties are unlikely to be obtained.

The sphericity of the silica-coated silicone rubber particle refers to the ratio between the length of the major axis and the length of the minor axis of one particle. The ratio closer to 1 is indicative of a shape closer to a true sphere, while a smaller ratio is indicative of a shape further deviated from a true sphere. The average of the sphericity is 0.8 or more. When the sphericity is less than 0.8, attributes necessary as a rubber particle, such as rubber physical properties, cannot be obtained, and sensory attributes such as the sense of looseness are not sufficient when the particle is used in cosmetics.

The hardness of the silicone rubber particle may also be important. The silicone rubber particle is required to have appropriate hardness in use applications such as in various cosmetics, a case when the particle is formulated in plastics to improve the properties, and a case when the particle is used for increasing the sliding properties between various sheets. Furthermore, not only the hardness of the rubber portion obtained by crosslinking the silicone rubber particle (i.e., the silicone component) for curing, but the hardness of the surface by the silica particle which coats the rubber particle may also be very important in various use applications.

Therefore, the ratio of the area covered with the silica particle in the entire surface area of the silicone rubber particle, that is, the coverage, is preferably high. Specifically, the coverage is preferably 70% or more. For increasing the coverage, it is preferable that the silica particle in the silica dispersed liquid prepared before emulsification be in a favorable dispersion state. If the coverage is less than 70%, the hardness of the surface of the silicone rubber particle is not sufficient. This also means that a particle being uniform in the spherical state and the particle diameter variation was not obtained in the process of generating the silicone rubber particle. Thus, sufficient properties cannot be obtained in the above-described various use applications. For achieving a larger coverage while the dispersion state of silica before emulsification and the condition of emulsification are constant, the use amount of the silica particle is preferably larger. When the use amount of the silica particle is constant, the coverage can be increased by lengthening the time of the emulsification process.

Sufficiently high sphericity indicates that the silica particle is uniformly dispersed on the surface of the silicone rubber particle. High sphericity can be achieved by performing curing for a sufficiently long time. Therefore, as the sphericity of the silicone rubber particle is higher, the hardness of the silicone rubber particle, especially the hardness of the surface of the silicone rubber particle, tends to be higher.

Also, as the time for curing is longer, the hardness of the silicone rubber particle, especially the hardness of the surface of the silicone rubber particle, tends to be higher.

The particle diameter of the silicone rubber particle of the present invention, when the particle is used in cosmetics, is desirably 100 µm or less. When the particle diameter is more than 100 µm, the powder becomes as large as it can be sensed by touch. Therefore, such a particle diameter is not preferable from the viewpoint of the impression of use.

The silica-coated silicone rubber particle is manufactured according to the method of claim 1.

The water dispersed body of the silica-coated rubber particle is obtained by dispersing the alkenyl siloxane of the component (B)-(I) and the organohydrogen polysiloxane of the component (B)-(II) in water in the presence of the silica particle of the component (A) and allowing a curing reaction to proceed as they are. Therefore, dispersion stability is excellent even when a so-called surfactant component is not used at all.

Although specific conditions are not defined as long as the method is within the above-described range, the following specific method is preferable for improving industrial stability and more advantageously controlling the shape and attributes of the particle.

That is, the oil-in-water emulsion includes
(A) 0.3 to 10 parts by mass of a silica particle, and
(B) 20 to 80 parts by mass of an organopolysiloxane including (I) an alkenyl group-containing organopolysiloxane in an amount of 19.95 to 79.95 parts by mass and (II) an organohydrogen polysiloxane in an amount of 0.05 to 59.05 parts by mass relative to the (I) alkenyl group-containing organopolysiloxane:
   (I) the alkenyl group-containing organopolysiloxane having an average composition formula represented by the general formula (1) and containing two or more alkenyl groups bonded to a silicon atom in one molecule,

      R¹ₐR²_{b}SiO_{(4-a-b)/2} (1)

      [wherein R¹ is the same or different monovalent hydrocarbon group not containing an aliphatic unsaturated group, R² is an alkenyl group, a is 0.999 to 2.999, b is 0.001 to 2, and a + b is 1 to 3],
   (II) the organohydrogen polysiloxane having an average composition formula represented by the general formula (2) and containing two or more hydrogen atoms bonded to a silicon atom in one molecule,

      R³_{C}SiO_{(4-c-d)/2} (2)

      [wherein R³ is the same or different monovalent hydrocarbon group not containing an aliphatic unsaturated group, c is 0.999 to 2.999, d is 0.001 to 2, and c + d is 1 to 3], and the silica-coated silicone rubber particle dispersed emulsion adds, to the oil-in-water emulsion, 1 to 5000 ppm of (C) the platinum group-based metal-containing addition catalyst relative to the total amount of the components (I) and (II).

### (Component (A))

The silica particle of the component (A) according to the present invention has on its surface a hydrophobized portion and a silanol group, and can be placed at the oil phase/water phase interface of the organopolysiloxane oil droplet of the component (B) in the emulsion water phase. Since such a silica particle has on its surface a silanol group as a hydrophilic group and a silanol group having been subjected to hydrophobization treatment at a prescribed ratio, the balance between hydrophilicity and hydrophobicity of the particle is controlled. Such a silica particle is placed at the oil phase/water phase interface including the organopolysiloxanes of (I) and (II) of the component (B), and exerts the emulsification function similar to the function possessed by known organic surfactants.

The silica particle of the component (A) is a particle of silicon dioxide produced by a synthesis process, and does not include mineral-based silicas such as diatomaceous earth and crystal quartz. Examples of the silicon dioxide produced by a synthesis process may include: fine powders by a dry process such as fumed silica, pyrogenic silica, and molten silica; and precipitated silica and colloidal silica by a wet process. These are known to those skilled in the art. Among these, pyrogenic silica, precipitated silica, and colloidal silica are preferable for use. The silica particle of (A) according to the present invention is hydrophilic silica on the surface of which a silanol group remains or hydrophobic silica on the surface of which a silanol group has been silylated. The hydrophobic silica can be produced by a known method of treating hydrophilic silica with halogenated organic silicon such as methyltrichlorosilane, alkoxysilanes such as dimethyldialkoxysilane, silazane, and low molecular weight methylpolysiloxane.

When a silica having on its surface a hydrophobized portion and a silanol group is used as the silica particle of the component (A), the surface tension of the silica particle falls within the region necessary for interfacial activity. Therefore, even when a common surfactant does not exist, the silica particle can be placed at the oil phase/water phase interface to be stabilized.

The ratio of the silanol remaining after silylation relative to the silanol group before silylation is preferably in the range of 50 to 95 mol%. When the remaining ratio of silanol is less than 50 mol% or more than 95 mol%, the function corresponding to the surfactant at the oil phase/water phase interface cannot be exerted. The silylation or the remaining ratio of silanol can be determined by measuring the carbon content through elemental analysis or measuring the remaining amount of a reactive silanol group on the surface of silica. The silica particle used for the preparation may include a particle of which the entire surface has been silylated or a particle of which the entire surface has not been silylated, as long as the ratio of silylation as a whole falls within the above-described range, and the necessary emulsification function can be expressed.

The carbon content of the suitably used silica particle of the component (A) having a hydrophobized portion and a silanol group is not limited as long as the object of functioning as a surfactant is achieved, but is preferably in the range of 0.1 to 10%. When the carbon content is less than 0.1% or more than 10%, a stable emulsion cannot be obtained. The carbon content is more preferably in the range of 0.1 to 10%.

The content of the component (A) in 100 parts by mass of the emulsion is preferably in the range of 0.3 to 10 parts by mass. When the content is less than 0.3 parts by mass, the stability of the water dispersion liquid deteriorates. When the content is more than 10 parts by mass, the coating of the rubber particle becomes excessive, causing unfavorable phenomena such as the sense of coarseness when used in cosmetics. The content of the component (A) is more preferably in the range of 1 to 7 parts by mass.

### (Component (B))

### (Component (I))

The component (I) is an organopolysiloxane having an average composition formula represented by the general formula (1) and containing two or more alkenyl groups bonded to silicon atoms in one molecule. The alkenyl group-containing organopolysiloxane of the component (I) is hereinafter also referred to as an alkenyl organopolysiloxane.

R¹ₐR²_{b}SiO_{(4-a-b)/2} (1)

In the formula (1), R¹ is the same or different monovalent hydrocarbon group not containing an aliphatic unsaturated group, R² is an alkenyl group, a is 0.999 to 2.999, b is 0.001 to 2, a + b is 1 to 3.

In the formula (1), R¹ preferably has 1 to 18 carbon atoms. It is preferable that R¹ is bonded with SiC-bond. Further, R¹ is preferably a substituted or unsubstituted hydrocarbon group having no aliphatic carbon-carbon multiple bond.

In the formula (1), R² preferably has 1 to 18 carbon atoms. Also, R² is preferably a monovalent hydrocarbon group having an aliphatic carbon-carbon multiple bond.

The alkenyl organopolysiloxane represented by the general formula (1) has an average of at least two R²s per molecule.

Examples of the alkenyl group in the component (I) may include alkenyl groups having 2 to 8 carbon atoms such as a vinyl group, an allyl group, a 1-butenyl group, and a 1-hexenyl group. The alkenyl group is preferably a vinyl group or an allyl group, and particularly preferably a vinyl group. These alkenyl groups react with the component (II) organohydrogen polysiloxane, which will be described later, to form a network structure. There are preferably two or more alkenyl groups in the molecule of the component (I). Such an alkenyl group may be bonded to the silicon atom at the terminal of the molecular chain or may be bonded to a silicon atom in the middle of the molecular chain.
From the viewpoint of the curing reaction rate, an alkenyl group-containing polyorganosiloxane in which an alkenyl group is bonded only to a silicon atom at the molecular chain terminal is preferable.

The other organic group bonded to the silicon atom in the component (I) is preferably a substituted or unsubstituted monovalent hydrocarbon group containing no aliphatic unsaturated bond having 1 to 12 carbon atoms. Specific examples of the other organic groups may include alkyl groups such as a methyl group, an ethyl group, a propyl group, an isopropyl group, a butyl group, an isobutyl group, a t-butyl group, a pentyl group, a neopentyl group, a hexyl group, a 2-ethylhexyl group, a heptyl group, an octyl group, a nonyl group, a decyl group, and a dodecyl group; cycloalkyl groups such as a cyclopentyl group, a cyclohexyl group, and a cycloheptyl group; aryl groups such as a phenyl group, a tolyl group, a xylyl group, a biphenyl group, and a naphthyl group; aralkyl groups such as a benzyl group, a phenylethyl group, a phenylpropyl group, and a methylbenzyl group; and substituted hydrocarbon groups, for example, those hydrocarbon groups in which a part or all of the hydrogen atoms in the group are substituted with a halogen atom, a cyano group, such as a chloromethyl group, a 2-bromoethyl group, a 3,3,3-trifluoropropyl group, a 3-chloropropyl group, a chlorophenyl group, a dibromophenyl group, a tetrachlorophenyl group, a difluorophenyl group, a β-cyanoethyl group, a γ-cyanopropyl group, and a β-cyanopropyl group. Particularly preferred organic groups are a methyl group and a phenyl group.

The component (I) may be linear or branched, or may be a mixture thereof. In the case of containing a branched alkenyl group-containing polyorganosiloxane, the crosslinking density becomes high, and thus the peeling force at low speed becomes heavier and it is difficult to reach the intended peeling force. Thus, a linear shape is more preferable. The alkenyl group-containing polyorganosiloxane is produced by a method known to those skilled in the art.

The viscosity of the component (I) at 25°C is preferably in the range of 5 to 2,000,000 mPa·s, more preferably 50 to 100,000 mPa·s, and particularly preferably 100 to 50,000 mPa·s. When the viscosity is less than 5 mPa·s or more than 2,000,000 mPa·s, emulsification is difficult and a stable aqueous dispersion cannot be obtained. Further, the preferable content of the component (I) used for obtaining the silicone rubber particle is in the range of 19.95 to 79.95 parts by mass. When the content is less than 19.95 parts by mass, sufficient emulsification accuracy cannot be obtained and the yield is lowered. When the content exceeds 79.95 parts by mass, the viscosity of the aqueous emulsion increases and handleability deteriorates. The content is more preferably in the range of 30 to 70 parts by mass.

The alkenyl group-containing polyorganosiloxane of the component (I) is produced by a method known to those skilled in the art, and can be produced by performing condensation and/or ring opening polymerization of cyclic low molecular weight siloxane using an acid catalyst such as sulfuric acid, hydrochloric acid, nitric acid, activated clay, or tris(2-chloroethyl)phosphite, or a base catalyst such as lithium hydroxide, sodium hydroxide, potassium hydroxide, tetramethylammonium hydroxide, tetra-n-butylammonium hydroxide, tetra-n-butylphosphonium hydroxide, sodium silanolate, and potassium silanolate.

### (Component (II))

An organohydrogenpolysiloxane of the component (II) is represented by the following average composition formula (2).

R³_{c}Sio_{(4-c-d)/2} (2)

In the formula (2), R³ is the same or different monovalent hydrocarbon group containing no aliphatic unsaturated group, c is 0.999 to 2.999, d is 0.001 to 2, and c + d is 1 to 3. As R³ in the formula (2), the hydrocarbon groups exemplified in the above R¹ are used. R³ in the formula (2) used is preferably an alkyl group, and more preferably a methyl group. The number of the hydrogen atom bonded to the silicon atom of the component (II) is preferably 2 or more in one molecule.

The organohydrogenpolysiloxane of the component (II) preferably contains 5% by mass or more of an organohydrogenpolysiloxane having a hydrogen atom bonded to a silicon atom in the molecular side chain.

The formulation amount of the component (II) in the composition according to the present invention is determined according to the amount of the alkenyl group in the component (I), and is adjusted such that the ratio (NH/NA) between the number (NA) of alkenyl groups bonded to a silicon atom in the component (I) and the number (NH) of hydrogen atoms bonded to a silicon atom contained in the component (II) satisfies 0.8 ≤ (NH/NA) ≤ 2.0, preferably 0.9 ≤ (NH/NA) ≤ 1.7. When NH/NA is less than 0.8, the curing of the composition does not sufficiently proceed, inhibiting the expression of the touch of looseness as a rubber particle. When NH/NA is 2.0 or more, the highly reactive organohydrogen polysiloxane remains in the rubber particle, raising a problem of safety as a material of cosmetics.

The organohydrogenpolysiloxane of the component (II) is also produced by a method known to those skilled in the art.

The viscosity of the component (II) is not particularly limited, but the viscosity at 25°C is preferably in the range of 1 to 3,000 mPa·s, and more preferably in the range of 1 to 1,500 mPa·s. If the viscosity is less than 1 mPa·s, the curability is too high and the rubber elasticity is insufficient. When the viscosity exceeds 3,000 mPa·s, the reactivity decreases and the curability becomes worse.

### (Component (C))

The component (C) is a platinum group-based catalyst which can be used as a hydrosilylation catalyst. The platinum group-based catalyst (C) includes metal or a compound containing the metal. Examples of the metal constituting the platinum group-based catalyst (C) may include platinum, rhodium, palladium, ruthenium, and iridium, with platinum being preferable. Alternatively, compounds containing these metals can be used. Among these, a platinum-based catalyst is highly reactive and thus suitable. Metal may be fixed to a carrier material having a fine particle shape (for example, activated carbon, aluminum oxide, and silicon oxide). Examples of the platinum compound may include platinum halides (for example, reaction products comprising PtCl₄, H₂PtCl₄·6H₂O, Na₂PtCl₄·4H₂O, H₂PtCl₄·6H₂O and cyclohexane), platinum-olefin complexes, platinum-alcohol complexes, platinum-alcoholate complexes, platinum-ether complexes, platinum-aldehyde complexes, platinum-ketone complexes, platinum-vinylsiloxane complexes (for example, platinum-1,3-divinyl 1,1,3,3-tetramethyldisiloxane complex, bis- (y-picoline)-platinum dichloride, trimethylene dipyridine-platinum dichloride, dicyclopentadiene-platinum dichloride, cyclooctadiene-platinum dichloride, cyclopentadiene-platinum dichloride), bis(alkynyl)bis(triphenylphosphine) platinum complexes, and bis(alkynyl)(cyclooctadiene)platinum complexes. Also, the hydrosilylation catalyst may be used in the microencapsulated form. In this case, example of a fine particle solid which contains a catalyst and is insoluble in polyorganosiloxane may include a thermoplastic resin (for example, a polyester resin or a silicone resin).

Also, the platinum-based catalyst can be used in the form of a clathrate compound, for example, in cyclodextrine.

For obtaining the silica-coated silicone rubber particle dispersed emulsion according to the present invention, it is preferable to add the platinum group-based catalyst of the component (C) such that the amount of the platinum group-based metal relative to the total weight of the components (I) and (II) as the diorganopolysiloxane of the component (B) becomes 1 to 5,000 ppm, preferably 5 to 1,000 ppm, and more preferably 20 to 500 ppm. When the content is less than 1 ppm, the time for curing may be extended, resulting in deteriorated production efficiency. When the content is more than 5,000 ppm, the appearance deteriorates, such as the coloring of the dispersed body into brown.

When the content is 500 ppm or more, the time during which the oil component to be dispersed can flow becomes short, with the result that the distortion of the particle when shear force has been applied for adding a catalyst remains as the shape of the particle. Thus, sliding properties exerted due to the shape close to a true sphere becomes unlikely to be obtained.

In curing, the dispersion of the silicone rubber particle is completed, thereafter the shape of the particle is fixed, and thereafter curing is completed. Since the sphericity improves as described above and the coverage of the surface by the silica particle also improves, the hardness of the surface increases. Thus, the properties suitable for various use applications can be obtained.

The method of preparing the dispersion emulsion of the silica-coated silicone rubber particles is not particularly limited, but it can be produced by a known method. The dispersion emulsion can be prepared by mixing the aforementioned (A), (B), and (C) using a conventional mixer suitable for preparing an emulsion, for example, a homogenizer, a colloid mill, a homomixer, a highspeed stator rotor agitator, and emulsifying it.

The silica-coated silicone rubber particle dispersed emulsion, may contain polyoxyalkylene alkyl ethers such as a polyoxyethylene tridecyl ether, a polyoxyethylene hexadecyl ether, and a polyoxyethylene octadecyl ether, nonionic surfactants such as a polyoxyethylene hydrogenated castor oil and a polyoxyethylene sorbitan acid ester, and ionic surfactants having a moderate skin irritancy such as sodium lauroyl glutamate and lysine sodium dilauroyl glutamate, in an amount that does not impair the object of the present invention.

The amount of the surfactant is 0.1 parts by mass or less, more preferably 0.06 parts by mass or less in 100 parts by mass of the emulsion.

Water is preferably used as the solvent that can be contained in the silica-coated silicone rubber particle dispersed emulsion of the present invention. Water is not particularly limited, but ion exchanged water is preferably used, and preferably has a pH of 2 to 12, and more preferably 4 to 10.

The silica-coated silicone rubber particle dispersed emulsion may contain, in addition to water, hydrophilic components such as ethanol, 1-propanol, 2-propanol, 1,2-propylene glycol, 1,2-butanediol, 1,3-butanediol, 1,5-pentanediol, 1,2-hexanediol, dipropylene glycol, glycerin, trimethylolpropane, and pentaerythritol in an amount that does not impair the object of the present invention.

The silica-coated silicone rubber particle dispersed emulsion may contain, as an antiseptic, salicylic acid, sodium benzoate, sodium dehydroacetate, potassium sorbate, phenoxyethanol, methyl parahydroxybenzoate, and butyl paraoxybenzoate, in an amount that does not impair the object of the present invention. In addition, depending on the use application, oil solutions such as a silicone oil as a lubricating component, and other components such as liquid paraffin, glycerin, and various perfumes, as a purpose, may be contained.

The method for isolating the silicone rubber particle from the silica-coated silicone rubber particle dispersed emulsion is not limited, and may be any known method. Examples of the method may include a process of removing water and concentrating by heating and/or decompression, and heating and drying the organic component, the trace component, and the like contained in the emulsion to obtain the silicone rubber particle. Alternatively, a spray drying process can be used. However, in terms of the prevention of the pyrolysis of the silicone rubber component, it is preferable to prevent the heating or drying for an extended time at high temperature, for example, at 200°C or higher.

### EXAMPLES

Next, the present invention will be described by way of examples. It is noted that the present invention is not limited by the examples. The method for preparing a dispersed emulsion of a silicone rubber particle which is coated with or is not coated with silica and isolating the silicone rubber particle from the emulsion in Examples and Comparative Examples was performed as follows. The storage stability, water dispersibility, and touch of the obtained silicone rubber particle dispersed emulsion, the coverage by the silica layer, sphericity, and blocking properties of the silicone rubber particle were measured, evaluated, or calculated as follows. Also, a formulation of a cosmetic was prepared as follows. The formulation amounts of the formulation of a cosmetic are shown in Table 1, the formulation amounts of Examples and Comparative Examples are shown in Table 2, the results of measurement and evaluation as an emulsion are shown in Table 3, and the results of measurement, evaluation, and calculation as a dried rubber particle are shown in Table 4.

### <Storage Stability Test>

Into a 50 ml screw vial, 30 g of the prepared silicone rubber particle dispersed emulsion was placed, and stored at room temperature for one month.
Thereafter, the presence or absence of creaming and sedimentation was checked.
Evaluation Criteria;
A: no creaming and sedimentation, B: a tendency of creaming and sedimentation, C: creaming and sedimentation observed

### <Water Dispersibility Test>

Into a 50 ml screw vial, 5 g of the prepared silicone rubber particle dispersed emulsion was placed, and 20 ml of deionized water was further added. Then, the screw vial was capped, and shaked by a hand 20 times. The dispersion state after the shaking was observed to evaluate water dispersibility.
Evaluation Criteria;
A: uniformly dispersed, B: partially dispersed, flocks remain, C: not dispersed

### <Touch Test>

On the back of the hand of three panelists, 0.05 g of the prepared silicone rubber particle dispersed emulsion (Examples 1 to 4, and Comparative Examples 1 to 4) and 0.03 g of the silicone rubber particle (Examples 1 to 4, and Comparative Examples 1 to 3) are placed, and applied by drawing a circle with an index finger.

The smoothness while being applied and the sense of looseness after having been applied and dried are evaluated.

The sense of looseness was evaluated by paired comparison, and the total of the evaluation points for each formulation was used for comparison. In the evaluation by paired comparison, 1 point was given to a formulation having the higher sense of looseness, 0 point to the lower, and 0.5 point to both having an identical sense of looseness.

On the basis of the total of the evaluation points by three panelists, each sample was evaluated as follows.
Evaluation criteria;
AA: 15 points and more, A: 9.0 to 13.5 points, B: 4.5 to 7.5 points, C: less than 3.0 points

### <Preparation of Formulation of Sun Protection Lotion>

The silicone rubber particle dispersed emulsion was poured into a water phase, or the dried silicone rubber particle was poured into an oil phase. Thus, a water-in-oil sun protection lotion was prepared according to the formulation indicated in Table 1. This formulation is defined as Formulation 1. For a comparison test, there was also prepared a formulation which does not contain both the rubber particle emulsion and the dried rubber particle.

The water phase and the oil phase were each mixed in a separate container while stirring at 3,000 rpm with an ULTRA TURRAX T 50 BASIC G45M manufactured by IKA. While the water phase was added in three parts to the prepared oil phase, the mixture was stirred and mixed at 3,000 rpm with an ULTRA TURRAX T 50 BASIC G45M.

### <Touch Test with Formulation of Sun Protection Lotion>

On the back of the hand of three panelists, 0.50 g of the prepared Formulation 1 is placed, and applied by drawing a circle with an index finger.

The smoothness while being applied and the sense of looseness after having been applied and dried are evaluated.

The sense of looseness was evaluated by paired comparison, and the total of the evaluation points for each formulation was used for comparison. In the evaluation by paired comparison, 1 point was given to a formulation having the higher sense of looseness, 0 point to a formulation having the lower sense of looseness, and 0.5 point to both having an identical sense of looseness.

On the basis of the total of the evaluation points by three panelists, each sample was evaluated as follows.
Evaluation criteria;
AA: 15 points and more, A: 9.0 to 13.5 points, B: 4.5 to 7.5 points, C: less than 3.0 points

**[Table 1]**

| <Formulation amount (unit: parts by mass)> | | | | |
|---|---|---|---|---|
| | | When rubber particle emulsion is formulated | When dried rubber particle is formulated | When both rubber particle emulsion and dried rubber particle are not formulated |
| Oil layer | Cyclopentasiloxane, dimethiconol | 2.0 | 2.0 | 2.0 |
| | Cyclopentasiloxane | 28.9 | 23.9 | 28.9 |
| | Dried rubber particle | - | 5.0 | - |
| | Distearyldimonium hectorite, denatured alcohol | 3.0 | 3.0 | 3.0 |
| | Ethylhexyl methoxycinnamate | 10.0 | 10.0 | 10.0 |
| | Ethylhexyl salicylate | 1.0 | 1.0 | 1.0 |
| | Cyclopentasiloxane, Caprylyl dimethicone ethoxy glucoside | 2.0 | 2.0 | 2.0 |
| | PEG/PPG-20/20 Phenylisopropyl Caprylyl dimethicone | 0.6 | 0.6 | 0.6 |
| Water layer | Butylene glycol | 2.5 | 2.5 | 2.5 |
| | Water | 40.0 | 50.0 | 50.0 |
| | Rubber particle emulsion | 10.0 | - | - |
| Total | | 100.0 | 100.0 | 100.0 |

### <Calculation Method for Coverage of Particle Surface by Silica>

The particle diameter of the isolated silicone rubber particle is observed through an electron microscope or an optical microscope. From the average value for the observed diameters and 0.9 as the specific gravity of silicone rubber, the surface area is calculated. The number of kg of the silica particle per unit square meter is calculated by dividing the amount of the silica particle used for preparing the silicone rubber particle dispersed emulsion by the value of the surface area of the silicone rubber particle.

### <Measurement Method for Sphericity of Particle>

The isolated silicone rubber particle sample is observed through an electron microscope or an optical microscope to measure the minor diameter and the major diameter. The ratio of minor diameter/major diameter is defined as sphericity. The ratio between the minor diameter and the major diameter is obtained by randomly placing the obtained particle sample on a sample board, taking a picture of the sample, measuring each of 50 spherical granules for the length (major diameter) of its major axis and the length (minor diameter) of a minor axis orthogonal to the midpoint of a major axis, calculating the ratio of the minor diameter to the major diameter for each granule, and obtaining the average of the obtained 50 ratio values.

### <Blocking Properties Test>

The isolated silicone rubber particle dispersed emulsion is dried at 50°C for 6 hours to evaporate moisture.

The obtained silicone rubber particle is put into a plastic syringe having an inner diameter of 5 mm, and compressed at a load of 500 g for one minute.

A powder is removed from the syringe, and the state of the removed powder is observed.
Evaluation Criteria;
AA: no lumps, A: some lumps, B: mostly lumps, C: only lumps observed

### <Example 1>

An oil-in-water silicone emulsion 1 was prepared as below. First, 5 parts by mass of a silica particle (A) was dispersed in 45 parts by mass of water by stirring the mixture at 4,000 rpm using an ULTRA TURRAX T50 basic shaft generator G45M manufactured by IKA to prepare a water dispersion liquid of silica particles. Next, in another container, 47.9 parts by mass of a vinyl group-containing polydimethylsiloxane (I) of which both terminals were blocked with a dimethylvinylsilyl group and the viscosity was 200 mPa·s (25°C), and 2.1 parts by mass of a methyl hydrogen polysiloxane (II) having an SiH group at the side chain in which both terminals of the molecular chain were blocked with a trimethylsiloxy group and the viscosity was 65 mPa·s (25°C) were mixed. Thus, a mixed oil in which the ratio NH/NA between the number (NA) of alkenyl groups and the number (NH) of hydrogen atoms bonded to a silicon atom was 1.2/1 was prepared. Next, the prepared mixed oil was added to the water dispersion liquid of silica particles under stirring at 4,000 rpm thereby to obtain an oil-in-water silicone emulsion (oil-in-water silicone emulsion 1). While the obtained silicone emulsion 1 was stirred at 200 rpm with a THREE-ONE MOTOR/propeller type stirring blade, 0.4 parts by mass of a platinum-vinylsiloxane complex solution (C) containing 1% by mass of a platinum atom was added. The mixture was stirred for 10 minutes to emulsify the platinum catalyst, so that it was dispersed in the particle. Thereafter, the shape of the particle was fixed over 10 minutes, and the curing was completed over another 10 minutes. Thus, a silicone rubber particle dispersed emulsion was obtained.

The particle diameter of the obtained silicone rubber particle dispersed emulsion was measured by a Beckman Coulter LS230 (manufactured by Beckman Coulter, Inc.). As a result, it was confirmed that the water dilution properties in preparing the measured liquid (the preparation of an aqueous solution having a solid content of about 10% by mass) was easy, and the water dispersibility was favorable. The median diameter (d50) was 11 µm, and the particle size distribution was narrow.

The obtained emulsion was dried at 150°C for one hour to remove water for isolation. Thus, a dried silicone rubber particle was obtained. The obtained silica-coated silicone rubber particle included a silicone rubber particle and a silica coating layer entirely or partially coating the surface of the silicone rubber particle.

### <Example 2>

A silicone rubber particle dispersed emulsion and a silicone rubber particle were obtained in the same manner as that in Example 1 except that, in the silicone emulsion 1 in Example 1, 49.0 parts by mass of the vinyl group-containing polydimethylsiloxane (I) of which viscosity was 1000 mPa·s (25°C), and 1.0 part by mass of the methyl hydrogen polysiloxane (II) were mixed to prepare a mixed oil in which the ratio NH/NA between the number (NA) of alkenyl groups and the number (NH) of hydrogen atoms bonded to a silicon atom was 1.5/1. The obtained silica-coated silicone rubber particle included a silicone rubber particle and a silica coating layer entirely or partially coating the surface of the silicone rubber particle.

The particle diameter of the obtained silicone rubber particle dispersed emulsion was measured by a Beckman Coulter LS230 (manufactured by Beckman Coulter, Inc.). As a result, it was confirmed that the median diameter d50 was 11 µm.

### <Example 3>

A silicone rubber particle dispersed emulsion and a silicone rubber particle were obtained in the same manner as that in Example 1 except that the amount of the silica particle of the component (A) in the silicone emulsion 1 in Example 1 was 3.0 parts by mass with the remainder being water. The obtained silica-coated silicone rubber particle included a silicone rubber particle and a silica coating layer entirely or partially coating the surface of the silicone rubber particle.

The particle diameter of the obtained silicone rubber particle dispersed emulsion was measured by a Beckman Coulter LS230 (manufactured by Beckman Coulter, Inc.). As a result, it was confirmed that the median diameter d50 was 11 µm.

### <Example 4>

A silicone rubber particle dispersed emulsion and a silicone rubber particle were obtained in the same manner as that in Example 1 except that the amount of the silica particle of the component (A) in the silicone emulsion 1 in Example 1 was 2.0 parts by mass with the remainder being water. The obtained silica-coated silicone rubber particle included a silicone rubber particle and a silica coating layer entirely or partially coating the surface of the silicone rubber particle.

The particle diameter of the obtained silicone rubber particle dispersed emulsion was measured by a Beckman Coulter LS230 (manufactured by Beckman Coulter, Inc.). As a result, it was confirmed that the median diameter d50 was 11 µm.

### <Comparative Example 1>

A silicone rubber particle dispersed emulsion and a silicone rubber particle were obtained in the same manner as that in Example 1 except that the amount of the silica particle of the component (A) in the silicone emulsion 1 in Example 1 was 15.0 parts by mass, and the amount of water was 37 parts by mass. The obtained silica-coated silicone rubber particle included a silicone rubber particle and a silica coating layer entirely or partially coating the surface of the silicone rubber particle.

The particle diameter of the obtained silicone rubber particle dispersed emulsion was measured by a Beckman Coulter LS230 (manufactured by Beckman Coulter, Inc.). As a result, it was confirmed that the median diameter d50 was 5 µm.

### <Comparative Example 2>

A silicone rubber particle dispersed emulsion and a silicone rubber particle were obtained in the same manner as that in Example 1 except that the amount of the silica particle of the component (A) in the silicone emulsion 1 in Example 1 was 0.2 parts by mass with the remainder being water. The obtained silica-coated silicone rubber particle included a silicone rubber particle and a silica coating layer entirely or partially coating the surface of the silicone rubber particle.

The particle diameter of the obtained silicone rubber particle dispersed emulsion was measured by a Beckman Coulter LS230 (manufactured by Beckman Coulter, Inc.). As a result, it was confirmed that the median diameter d50 was 100 µm.

### <Comparative Example 3>

A silicone rubber particle dispersed emulsion and a silicone rubber particle were obtained in the same manner as that in Example 1 except that, in the silicone emulsion 1 in Example 1, the amount of the vinyl group-containing polydimethylsiloxane (I) was 67.1 parts by mass, the amount of the methyl hydrogen polysiloxane (II) was 2.9 parts by mass, and the amount of polyoxyethylene cetyl ether (ethyleneoxide added molar number: 13) instead of the silica particle (A) was 1.0 part by mass with the remainder being water. The particle diameter of the obtained silicone rubber particle dispersed emulsion was measured by a Beckman Coulter LS230 (manufactured by Beckman Coulter, Inc.). As a result, it was confirmed that the median diameter d50 was 6 µm.

### <Comparative Example 4>

A silicone rubber particle dispersed emulsion and a silicone rubber particle were obtained in the same manner as that in Example 1 except that, in the silicone emulsion 1 in Example 1, the amount of polyoxyethylene cetyl ether (ethyleneoxide added molar number 13) instead of the silica particle (A) was 1.0 part by mass with the remainder being water. In the preparation of Formulation 1 in Comparative Example 4, they were added as an emulsion but not as a dry particle.

The particle diameter of the obtained silicone rubber particle dispersed emulsion was measured by a Beckman Coulter LS230 (manufactured by Beckman Coulter, Inc.). As a result, it was confirmed that the median diameter d50 was 5 µm.

### <Comparative Example 5>

In preparation of Formulation 1, a blank formulation not blended as rubber particle emulsion or dry rubber particles was prepared and subjected to the touch test. This is referred to as Comparative Example 5.

**[Table 2]**

| Formulation amount (unit: parts by mass) | | | | | | | | |
|---|---|---|---|---|---|---|---|---|
| | Example 1 | Example 2 | Example 3 | Example 4 | Comparative Example 1 | Comparative Example 2 | Comparative Example 3 | Comparative Example 4 |
| Vinyl group-containing polydimethylsiloxane (viscosity: 200 mPa·s (25°C)) | 47.9 | | 47.9 | 47.9 | 46.8 | 47.9 | 67.1 | 47.9 |
| Vinyl group-containing polydimethylsiloxane (viscosity: 1,000 mPa·s (25°C)) | | 49.0 | | | | | | |
| Methyl hydrogen polysiloxane (viscosity: 65 mPa·s (25°C)) | 2.1 | 1.0 | 2.1 | 2.1 | 1.2 | 2.1 | 2.9 | 2.1 |
| Silica particle | 5.0 | 5.0 | 3.0 | 2.0 | 15.0 | 0.2 | | |
| Polyoxyethylene cetyl ether E.O. 13 mol | | | | | | | 1.0 | 1.0 |
| Water | 45.0 | 45.0 | 47.0 | 48.0 | 37.0 | 49.8 | 29.0 | 49.0 |
| Total | 100.0 | 100.0 | 100.0 | 100.0 | 100.0 | 100.0 | 100.0 | 100.0 |
| Number of alkenyl groups/number of hydrogen atoms bound to silicon atom (NH/NA) | 1.2 | 1.5 | 1.2 | 1.2 | 1.2 | 1.2 | 1.2 | 1.2 |

**[Table 3]**

| <Evaluation result of silicone particle dispersed emulsion> | | | | | | | | | |
|---|---|---|---|---|---|---|---|---|---|
| | Example 1 | Example 2 | Example 3 | Example 4 | Comparative Example 1 | Comparative Example 2 | Comparative Example 3 | Comparative Example 4 | Comparative Example 5 |
| Storage stability | A | A | A | A | A | C | A | C | - |
| Dispersibility in water | A | A | A | A | A | A | C | B | - |
| Sense of touch as emulsion | AA | A | AA | AA | C | B | B | A | - |
| Sense of touch as sun protection lotion (Formulation 1) | A | A | A | A | B | B | C(dispersion failure) | C(dispersion failure) | C |

**[Table 4]**

| <Calculation, measurement, and evaluation result of dried silicone rubber particle> | | | | | | | |
|---|---|---|---|---|---|---|---|
| | Example 1 | Example 2 | Example 3 | Example 4 | Comparative Example 1 | Comparative Example 2 | Comparative Example 3 |
| Coating amount of silica layer (kg/m²) | 1.6 × 10⁻⁷ | 1.6 × 10⁻⁷ | 1.0 × 10⁻⁷ | 7.7 × 10⁻⁸ | 3.4 × 10⁻⁶ | 1.3 × 10⁻⁹ | - |
| Sphericity (minor diameter/major diameter) | > 0.95 | > 0.95 | > 0.95 | > 0.95 | > 0.95 | > 0.95 | 0.78 |
| Blocking properties | A | A | A | A | A | A | C |
| Sense of touch as particle single substance | A | A | AA | AA | C | B | - |
| Sense of touch as sun protection lotion (Formulation 1) | A | A | AA | AA | C | B | C to B |

### INDUSTRIAL APPLICABILITY

The silica-coated silicone rubber particle according to the present invention has a surface sufficiently coated with a silica particle, and exhibits high sphericity. Therefore, the physical properties favorable as a rubber particle can be exerted without raising problems which are likely to occur in a typical silicone particle, such as blocking. Consequently, the silica-coated silicone rubber particle according to the present invention can be employed for many use applications such as cosmetics and various industrial uses. Also, since an organic surfactant is not used, the associated environmental problem is solved, and stable manufacture is enabled. Furthermore, the silica-coated silicone rubber particle manufactured according to the present invention can exist in the form of a stable emulsion, and can be advantageously used in many industries.

## Claims

1. A method for manufacturing a silica-coated silicone rubber particle, the silica-coated silicone rubber particle including a silicone rubber particle, and a coating layer configured to coat a surface of the silicone rubber particle and be formed from a silica particle, wherein a coating amount of the coating layer is 2.0 × 10⁻⁹ to 3.2 × 10⁻⁶ kg/m², and the silicone rubber particle has a sphericity of 0.8 to 1.0, the method comprising:
emulsifying in water
(A) a silica particle, selected from the group of hydrophilic silica on the surface of which a silanol group remains or hydrophobic silica on the surface of which a silanol group has been silylated and
(B) an organopolysiloxane including
(I) an alkenyl group-containing organopolysiloxane, and
(II) an organohydrogen polysiloxane to prepare an oil-in-water emulsion;
thereafter adding, to the oil-in-water emulsion,
(C) a platinum group-based metal-containing addition reaction catalyst
to initiate a hydrosilylation reaction of the components (I) and (II) for curing, wherein when it is cured, without adding any nonionic, anionic, cationic, and amphoteric organic surfactant, dispersion of the silicone rubber particle is completed, thereafter the shape of the particle is fixed, and thereafter curing is completed; and
removing the water from the emulsion.

## Patentansprüche

1. Verfahren zur Herstellung eines siliciumdioxidbeschichteten Siliconkautschukpartikels, wobei das siliciumdioxidbeschichtete Siliconkautschukpartikel ein Siliconkautschukpartikel und eine Beschichtungsschicht, die dafür gestaltet ist, eine Oberfläche des Siliconkautschukpartikels zu beschichten und aus einem Siliciumdioxidpartikel gebildet zu werden, umfasst, wobei die Beschichtungsmenge der Beschichtungsschicht 2,0 x 10⁻⁹ bis 3,2 x 10⁻⁶ kg/m² beträgt und das Siliconkautschukpartikel eine Sphärizität von 0,8 bis 1,0 aufweist, wobei das Verfahren umfasst:
Emulgieren in Wasser von
(A) einem Siliciumdioxidpartikel ausgewählt aus der Gruppe von hydrophilem Siliciumdioxid auf der Oberfläche, von der eine Silanolgruppe verbleibt, oder hydrophobem Siliciumdioxid auf der Oberfläche, von der eine Silanolgruppe silyliert worden ist, und
(B) einem Organopolysiloxan umfassend
(I) ein Alkenylgruppe-enthaltendes Organopolysiloxan und
(II) ein Organohydrogenpolysiloxan,
um eine Öl-in-Wasser-Emulsion zu erzeugen;
anschließend zu der Öl-in-wasser-Emulsion Zugeben von
(C) einem metallhaltigen Additionsreaktionskatalysator auf Platingruppenbasis,
um eine Hydrosilylierungsreaktion der Komponenten(I)und (II)zum Härten auszulösen, wobei, wenn gehärtet ist, die Dispersion des Siliconkautschukpartikels ohne Zugabe eines nichtionischen, anionischen, kationischen oder amphoteren organischen grenzflächenaktiven Mittels abgeschlossen ist, anschließend die Form des Partikels fixiert wird und anschließend das Härten abgeschlossen ist; und
Entfernen des Wassers aus der Emulsion.

## Revendications

1. Procédé pour la fabrication d'une particule de caoutchouc de silicone revêtue par de la silice, la particule de caoutchouc de silicone revêtue par de la silice comportant une particule de caoutchouc de silicone, et une couche de revêtement conçue pour revêtir une surface de la particule de caoutchouc de silicone et pour être formée à partir d'une particule de silice, une quantité de revêtement de la couche de revêtement étant de 2,0 x 10⁻⁹ à 3,2 x 10⁻⁶ kg/m², et la particule de caoutchouc de silicone possédant une sphéricité de 0,8 à 1,0, le procédé comprenant:
l'émulsification dans l'eau
(A) d'une particule de silice, choisie dans le groupe de la silice hydrophile sur la surface de laquelle reste un groupe silanol ou de la silice hydrophile sur la surface de laquelle un groupe silanol a été silylé et
(B) d'un organopolysiloxane comportant
(I) un organopolysiloxane contenant un groupe alcényle, et
(II) un polysiloxane organohydrogéné pour préparer une émulsion huile-dans-eau ;
l'ajout subséquent, à l'émulsion huile-dans-eau,
(C) d'un catalyseur de réaction d'addition contenant un métal à base du groupe du platine pour initier une réaction d'hydrosilylation des composants (I) et (II) pour le durcissement, dans lequel lorsqu'elle est durcie, sans ajout d'un quelconque tensioactif organique non ionique, anionique, cationique, et amphotère, la dispersion de la particule de caoutchouc de silicone est terminée, subséquemment la forme de la particule est fixée, et subséquemment le durcissement est terminé ; et
l'élimination de l'eau de l'émulsion.
